# EUROPEAN PATENT SPECIFICATION

(11) **EP 4 337 644 B1**
(45) Date of publication and mention of the grant of the patent: **26.02.2025**
(21) Application number: 22728138.3
(22) Date of filing: 06.05.2022
(51) Int. Cl.: C07D 257/06, C07D 271/113, A01P 13/02, A01N 43/713, A01N 43/82

(54) **HERBICIDAL COMPOUNDS**
HERBIZIDE VERBINDUNGEN
COMPOSÉS HERBICIDES

(30) Priority: 14.05.2021 GB 202106945
(43) Date of publication of application: 20.03.2024
(73) Proprietor: Syngenta Crop Protection AG, 4058 Basel (CH)
(72) Inventor: BURTON, Paul Matthew, Bracknell, RG42 6EY (GB); EMERY, Katie, Bracknell, RG42 6EY (GB)
(74) Representative: SYNGENTA IP
(86) International application number: PCT/EP2022/062250
(87) International publication number: WO 2022/238247

(56) References cited:
- WO-A1-2012/028579
- WO-A1-2012/126932
- WO-A1-2017/102275
- WO-A1-2018/177871
- WO-A1-2019/122345

## Description

The present invention relates to novel herbicidal compounds, to processes for their preparation, to herbicidal compositions which comprise the novel compounds, and to their use for controlling weeds, in particular in crops of useful plants, or for inhibiting plant growth.

N-(tetrazol-5-yl)- and N-(1,3,4-oxadiazol-2-yl) arylcarboxamides are disclosed in, for example, WO2012/028579 and WO2012/126932 respectively. The present invention relates to novel arylcarboxamides.

Thus, according to the present invention there is provided a compound of Formula (I): 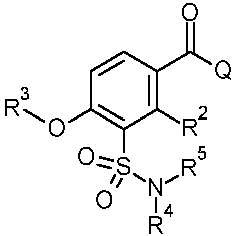
or an agronomically acceptable salt thereof,
wherein: -
   Q is Q¹ or Q²; 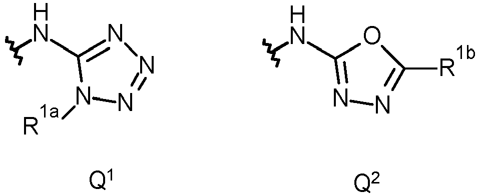
   R^{1a} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
   R^{1b} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
   R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
   R³ is C₁-C₃haloalkyl;
   R⁴ is selected from the group consisting of C₁-C₆alkyl-, C₁-C₆haloalkyl-, C₃-C₆cycloalkyl-, C₃-C₆cycloalkyl-C₁-C₃alkyl-, C₁-C₃alkoxy-C₁-C₃alkyl-, C₁-C₄haloalkoxy-C₁-C₄alkyl-, C₁-C₄alkoxy-C₁-C₄haloalkyl-, C₁-C₃alkyl-S(O)ₚC₁-C₃alkyl-, and cyano-C₁-C₆alkyl;
   R⁵ is selected from the group consisting of hydrogen, C₁-C₆alkyl-, C₁-C₆haloalkyl and C₁-C₆cycloalkyl; or
   R⁴ and R⁵ together with the nitrogen atom to which they are attached form an optionally substituted 5- or 6-membered saturated heterocycle; and
   p is 0, 1 or 2.

C₁-C₆alkyl and C₁-C₄alkyl groups include, for example, methyl (Me, CH₃), ethyl (Et, C₂H₅), n-propyl (n-Pr), isopropyl (i-Pr), n-butyl (n-Bu), isobutyl (i-Bu), *sec*-butyl and *tert*-butyl (t-Bu).

C₃-C₆cycloalkyl- includes cyclopropyl (c-propyl (c-Pr)), cyclobutyl (c-butyl (c-Bu)), cyclopentyl (c-pentyl) and cyclohexyl (c-hexyl).

C₁-C₃alkoxy- includes, for example, methoxy- and ethoxy-.

Halogen (or halo) encompasses fluorine, chlorine, bromine or iodine. The same correspondingly applies to halogen in the context of other definitions, such as haloalkyl.

C₁-C₆haloalkyl includes, for example, fluoromethyl-, difluoromethyl-, trifluoromethyl-, chloromethyl-, dichloromethyl-, trichloromethyl-, 2,2,2-trifluoroethyl-, 2,2-difluoroethyl, 1,1-difluoroethyl, 1,1,2,2-tetrafluoroethyl, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl-, heptafluoro-n-propyl and perfluoro-n-hexyl. C₁-C₄haloalkyl includes, for example, fluoromethyl-, difluoromethyl-, trifluoromethyl-, chloromethyl-, dichloromethyl-, trichloromethyl-, 2,2,2-trifluoroethyl-, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl- and heptafluoro-n-propyl-.

C₁-C₄alkoxy-C₁-C₄alkyl- includes, for example, methoxyethyl-.

C₁-C₄haloalkoxy-C₁-C₄alkyl- includes, for example, trifluoromethoxyethyl-.

C₁-C₆alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In one embodiment of the present invention, Q is Q¹. Thus, in this embodiment the compound of Formula (I) is a compound of Formula (Ia): 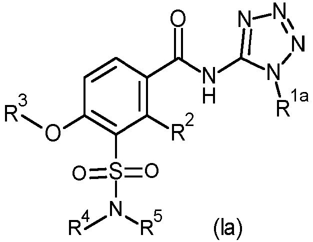 wherein R^{1a}, R², R³, R⁴ and R⁵ are as defined with regard to a compound of Formula (I).

In another embodiment of the present invention, Q is Q². Thus, in this particular embodiment of the present invention there is provided a compound of Formula (Ib) 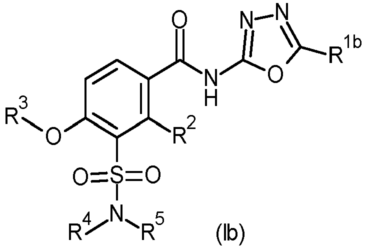 wherein R^{1b}, R², R³, R⁴ and R⁵ are as defined with regard to a compound of Formula (I).

In a preferred embodiment of the present invention, R^{1a} and R^{1b} are selected from the group consisting of methyl, ethyl and n-propyl.

In a preferred embodiment of the present invention, R² is selected from the group consisting of methyl, Cl, -CF₃ and -SO₂methyl, more preferably Cl.

In another preferred embodiment of the present invention, R³ is -CF₃ or -CHF₂.

In one embodiment of the present invention, R⁴ is preferably selected from the group consisting of hydrogen, methyl, ethyl and cyclopropyl-CH₂-.

In another embodiment of the present invention R⁵ is hydrogen or methyl.

In another embodiment of the present invention R⁴ and R⁵ taken together form a 5- or 6-membered saturated heterocycle which is optionally oxo substituted. In another embodiment of the invention R⁴ and R⁵ taken together form a 5- or 6-membered saturated heterocycle which is optionally oxo substituted selected from the group consisting of -C(O)-CH₂CH₂CH₂CH₂-, - CH₂CH₂OCH₂CH₂-, -C(O)CH₂CH₂CH₂-, -CH₂CH₂CH₂CH₂CH₂- and -CH₂CH₂CH₂CH₂-, preferably -C(O)-CH₂CH₂CH₂CH₂-.

Compounds of Formula (I) (and certain intermediate compounds used to synthesise compound of Formula (I)) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also includes all possible geometric and tautomeric forms of a compound of formula (I).

The present invention also includes agronomically acceptable salts that the compounds of Formula (I) may form with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used as salt formers, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

The compounds of Formula (I) according to the invention can be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound of the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate, prop-2-ynyl 4-amino-3-chloro-5-fluoro-6-(7-fluoro-1H-indol-6-yl)pyridine-2-carboxylate and cyanomethyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl 2-[[3-[[3-chloro-5-fluoro-6-[3-methyl-2,6-dioxo-4-(trifluoromethyl)pyrimidin-1-yl]-2-pyridyl]oxy]acetate and 6-chloro-4-(2,7-dimethyl-1-naphthyl)-5-hydroxy-2-methyl-pyridazin-3-one.

The mixing partners of the compound of Formula I may also be in the form of esters or salts, as mentioned e.g. in The Pesticide Manual, Sixteenth Edition, British Crop Protection Council, 2012.

The compound of Formula I can also be used in mixtures with other agrochemicals such as fungicides, nematicides or insecticides, examples of which are given in The Pesticide Manual.

The mixing ratio of the compound of Formula I to the mixing partner is preferably from 1: 100 to 1000: 1.

The mixtures can advantageously be used in the above-mentioned formulations (in which case "active ingredient" relates to the respective mixture of compound of Formula I with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil. Particularly preferred are mixtures of a compound of Formula I with cyprosulfamide, isoxadifen-ethyl, cloquintocet-mexyl and/or metcamifen.

The safeners of the compound of Formula I may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula I to safener is from 100:1 to 1:10, especially from 20:1 to 1:1.

The mixtures can advantageously be used in the above-mentioned formulations (in which case "active ingredient" relates to the respective mixture of compound of Formula I with the safener).

The present invention still further provides a method of controlling weeds at a locus said method comprising application to the locus of a weed controlling amount of a composition comprising a compound of Formula (I). Moreover, the present invention further provides a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. Generally, the plants to be controlled are unwanted plants (weeds). 'Locus' means the area in which the plants are growing or will grow. Some crop plants may be inherently tolerant to herbicidal effects of compounds of Formula (I). However, in some instances tolerance may need to be engineered into the crop plant, for example by way of genetic engineering. Thus, it is possible that the crop plant is rendered tolerant to HPPD-inhibitors via genetic engineering. Methods of rending crop plants tolerant to HPPD-inhibitors are known, for example from WO0246387. Thus in an even more preferred embodiment the crop plant is transgenic in respect of a polynucleotide comprising a DNA sequence which encodes an HPPD-inhibitor resistant HPPD enzyme derived from a bacterium, more particularly from *Pseudomonas fluorescens* or *Shewanella colwelliana,* or from a plant, more particularly, derived from a monocot plant or, yet more particularly, from a barley, maize, wheat, rice, *Brachiaria, Cenchrus, Lolium, Festuca, Setaria, Eleusine, Sorghum or Avena* species. Several HPPD-tolerant soybean transgenic "events" are known and include for example SYHT04R (WO2012/082542), SYHT0H2 (WO2012/082548) and FG72. Other polynucleotide sequences that can be used to provide plants which are tolerant to the compounds of the present invention are disclosed in, for example, WO2010/085705 and WO2011/068567. Crop plants in which the composition according to the invention can be used thus include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled may be both monocotyledonous species, for example Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum, and dicotyledonous species, for example Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium. Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

The compounds of the present invention can be prepared according to the following schemes.

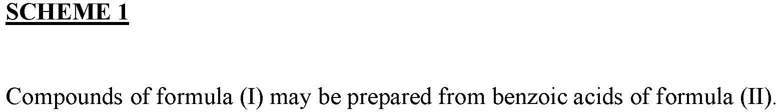

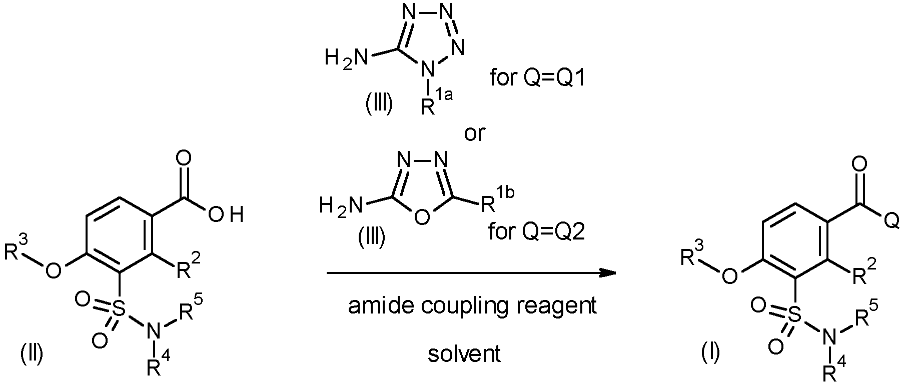

Where Q=Q1, benzoic acids of formula (II) are reacted with amines of formula (III). Where Q=Q2, benzoic acids of formula (II) are reacted with amines of formula (IV). In both cases, an amide coupling reagent is used. An example of a suitable amide coupling reagent is thionyl chloride. An example of a suitable solvent is 3-methylpyridine.

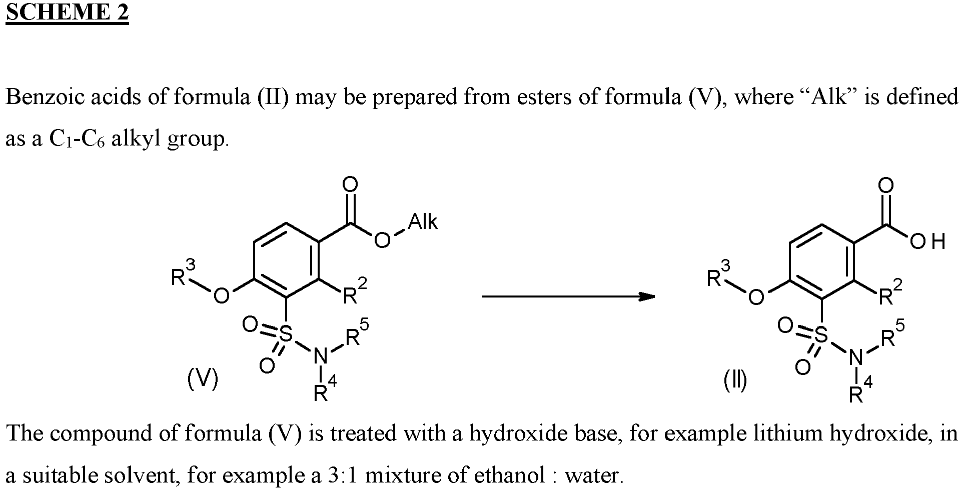

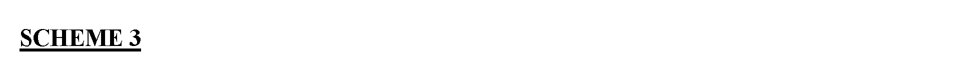

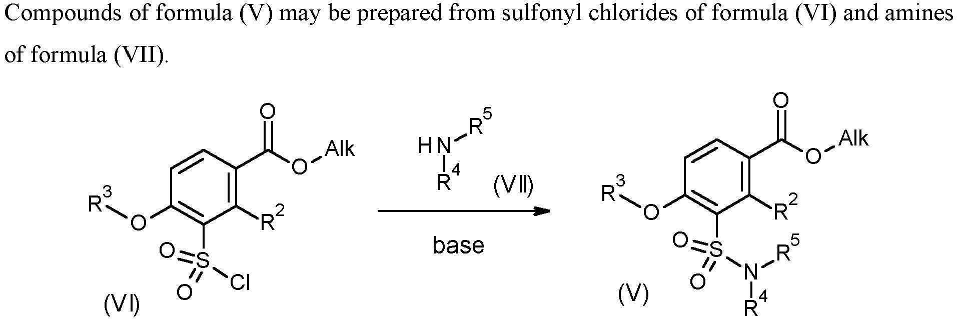

The compound of formula (VI) is treated with a compound of formula (VII) and a base, for example triethylamine, in a suitable solvent, for example acetonitrile.

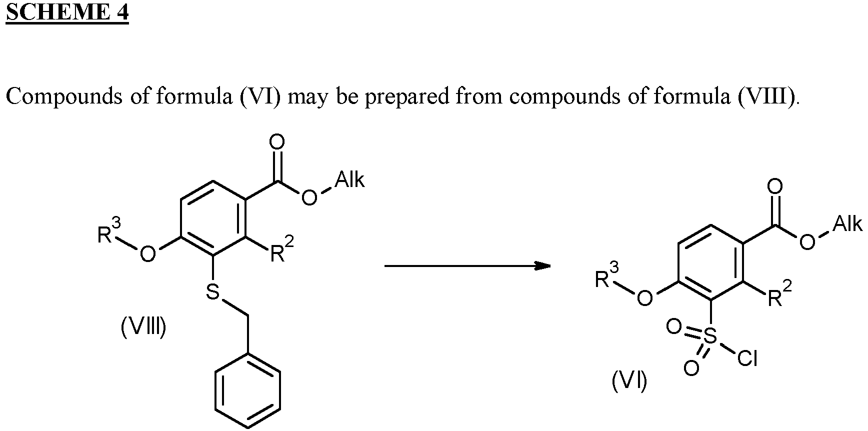

The compound of formula (VIII) is treated with a suitable oxidant, for example 1,3-dichloro-5,5-dimethyl-imidazolidine-2,4-dione.

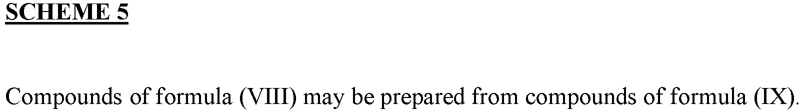

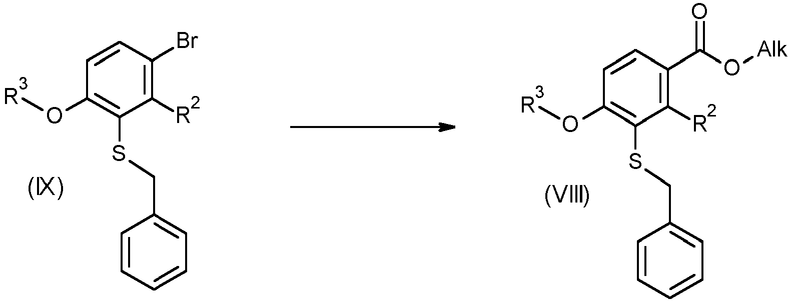

The compound of formula (IX) is reacted in a carbon monoxide atmosphere with a palladium catalyst, for example dichlorodiallyl palladium, with a suitable ligand, for example (+/-)-BINAP, with a suitable base, for example triethylamine. The solvent will depend on the identity of "Alk". For example, where "Alk" is methyl, the solvent used is methanol. For example, where "Alk" is ethyl, the solvent used is ethanol.

The method of synthesis of (IX) will depend on the nature of the R³ and R² groups.

For example, where R³ is CF₃ and R² is chloro, compounds of formula (IX) may be prepared from 1-bromo-2-chloro-4-(trifluoromethoxy)benzene and (benzyldisulfanyl)methylbenzene. 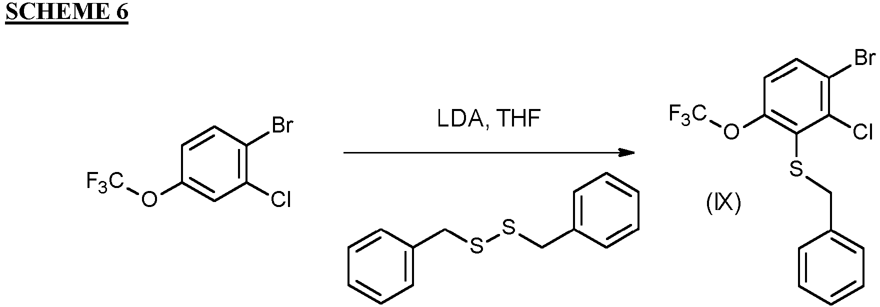 1-bromo-2-chloro-4-(trifluoromethoxy)benzene is treated with lithium diisopropylamide (LDA) in a suitable solvent, for example tetrahydrofuran, then reacted with (benzyldisulfanyl)methylbenzene.

Alternatively, compounds of Formula (IX) where R² is not chloro may be prepared from compounds of Formula (IX), where R² is chloro. For example, a compound of Formula (IX) where R² = chloro may be treated with [1,3-bis(2,6-diisopropylphenyl)imidazol-2-ylidene](3-chloropyridyl)palladium(II) dichloride, trimethyl boroxine and potassium carbonate, in a suitable solvent, for example 1,4-dioxane, to give the corresponding compound of Formula (IX) where R² is methyl. 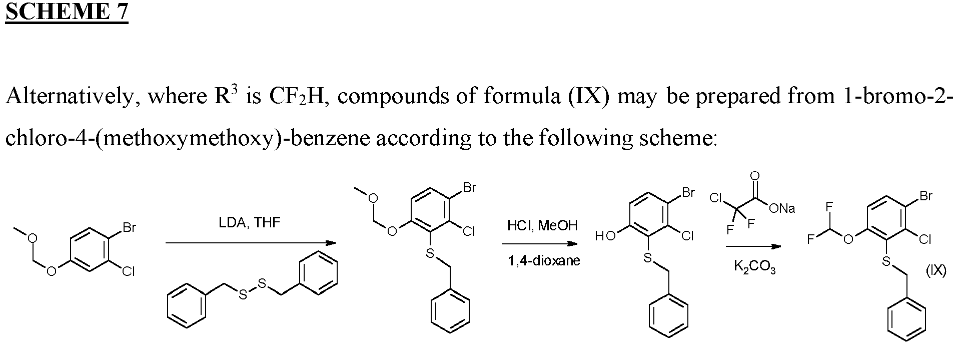 1-bromo-2-chloro-4-(methoxymethoxy)-benzene is treated with lithium diisopropylamide and (benzyldisulfanyl)methylbenzene to give 3-benzylsulfanyl-1-bromo-2-chloro-4-(methoxymethoxy)benzene. 3-benzylsulfanyl-1-bromo-2-chloro-4-(methoxymethoxy)benzene is in turn treated with HCl in methanol to give 2-benzylsulfanyl-4-bromo-3-chloro-phenol. 2-benzylsulfanyl-4-bromo-3-chloro-phenol is in turn treated with sodium 2-chloro-2,2-difluoroacetate and a base (for example potassium carbonate) to give the compound of formula (IX) where R³ is CF₂H and R² is chloro.

Thus according to the present invention there is still further provided a compound of Formula (II) 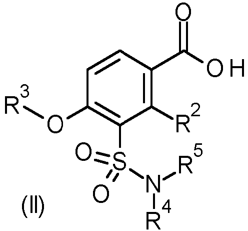 wherein R², R³, R⁴ and R⁵ are as defined in the compound of Formula (I) above.

The present invention still further provides a compound of Formula (V) 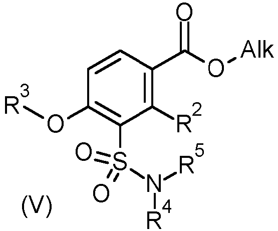 wherein "Alk" is C₁-C₆ alkyl and R², R³, R⁴ and R⁵ are as defined in the compound of Formula (I) above.

The following non-limiting examples provide specific synthesis methods for representative compounds of the present invention, as referred to in Table 1 provided herein.

### Example 1: Preparation of Compound 1.002

### Step 1: Preparation of 3-benzylsulfanyl-1-bromo-2-chloro-4-(trifluoromethoxy)benzene

To a 3 necked flask, equipped with a thermometer, containing tetrahydrofuran (200 mL) (that was purged and filled with N2) was added diisopropylamine (6.68 g, 9.30 mL, 65.3 mmol) . The reaction was stirred at -78 °C using a dry ice/acetone bath for 30 min. n-Butyllithium in hexane (2.5 M, 23 mL, 58.1 mmol) was added dropwise, maintaining the temperature at -78 °C and stirred for 40 min. A solution of 1-bromo-2-chloro-4-(trifluoromethoxy)benzene (10.0 g, 36.3 mmol) made up to 20 mL by addition of THF was added dropwise, maintaining the temperature at -78 °C and stirred for 2hr. Benzyldisulfanyl)methylbenzene (17.9 g, 72.6 mmol) in THF (30 mL) is added dropwise and the mixture was stirred at -78 °C for 1 h. Whilst still cold, the reaction mixture was quenched by adding it into a stirred solution of iced water. 2 M HCl was added to acidify the solution and followed by Ethyl acetate extraction x 3. The organic phase was concentrated in vacuo and purified by flash column chromatography (0-10% gradient of EtOAc in cyclohexane). Product-containing fractions were combined and partitioned with 1M NaOH, this added purification step removes the impurity phenylmethanethiol. The organics are again collected and concentrated in vacuo to afford 3-benzylsulfanyl-1-bromo-2-chloro-4-(trifluoromethoxy)benzene (8.5 g, 21 mmol, 56%) as a yellow oil. 1H NMR (CDCl₃): 7.59 (d, 1H), 7.25-7.18 (m, 5H), 7.05 (m, 1H), 4.09 (s, 2H)

### Step 2: Preparation of methyl 3-benzylsulfanyl-2-chloro-4-(trifluoromethoxy)benzoate

To a vessel charged with 3-benzylsulfanyl-1-bromo-2-chloro-4-(trifluoromethoxy)benzene (4.00 g, 10.0 mmol) was added methanol (67 mL), triethylamine (3.07 g, 30.2 mmol, 4.23 mL) and [(R)-BINAP Pd(allyl)]Cl (0.406 g, 0.503 mmol). Nitrogen was added to the vessel and the lid was sealed. The vessel was transferred to the Chemscan II and lines were connected and the system setup using 100 °C and 5 Bar as the reaction parameters. The reaction was set to stop when 3 hours had passed. Once the reaction had returned to ambient and the purge and venting cycles had successfully been completed the vessel was disconnected from the Chemscan II. The reaction mixture was concentrated in vacuo and was added EtOAc (100 mL), 2 M aq. HCl (80 mL) and brine (40 mL). The phases were separated and the aqueous phase was extracted with EtOAc (50 mL x 3). The combined organic phases were dried (MgSO4) and filtered. The filtrate was adsorbed onto silica and the crude product was purified by flash column chromatography, (0-10% gradient of EtOAc in cyclohexane). Product containing fractions were combined and concentrated in vacuo to afford methyl 3-benzylsulfanyl-2-chloro-4-(trifluoromethoxy)benzoate (2.01 g, 4.80 mmol, 48%) as a yellow oil. 1H NMR (400 MHz, Dichloromethane-d2): δ = 7.71 (d, J = 8.8 Hz, 1H), 7.29 - 7.17 (m, 6H), 4.09 (s, 2H), 3.91 (s, 3H).

### Step 3: Preparation of methyl 2-chloro-3-chlorosulfonyl-4-(trifluoromethoxy)benzoate

To a 3-necked flask containing 1,3-dichloro-5,5-dimethyl-imidazolidine-2,4-dione (2.09 g, 10.6 mmol) was added Acetonitrile (80 mL) and the reaction mixture was cooled to 0 °C. Water (1.1 mL) and Acetic acid (1.6 mL) were then added. A solution of methyl 3-benzylsulfanyl-2-chloro-4-(trifluoromethoxy)benzoate (2.0 g, 5.3 mmol) in acetonitrile (80 mL) was added over 10 min via a dropping funnel. The rate of addition was limited to keep the temperature below 10 °C. The reaction was warmed to RT. The reaction mixture was concentrated under reduced pressure with bath temperature of 30 °C. The residue was taken up in dichloromethane, and added to cooled (0 °C), saturated aqueous sodium bicarbonate. The organic phase was separated and dried (MgSO₄) and concentrated in vacuo to afford methyl 2-chloro-3-chlorosulfonyl-4-(trifluoromethoxy)benzoate as a yellow oil. The crude product is carried forward without further purification.

### Step 4: Preparation of methyl 2-chloro-3-(ethylsulfamoyl)-4-(trifluoromethoxy)benzoate

To a flask containing a solution of methyl 2-chloro-3-chlorosulfonyl-4-(trifluoromethoxy)benzoate (0.936 g, 2.65 mmol) in acetonitrile (50 mL) was added ethylamine (2.65 mL, 5.30 mmol), then triethylamine (0.739 mL, 5.30 mmol). The reaction mixture was stirred at RT overnight. The reaction was concentrated in vacuo and quenched by addition of 2 M aq. HCl (100 mL) and extracted with ethyl acetate x 3. The combined organic phases were dried (MgSO₄), filtered and concentrated in vacuo and purified by chromatography, (0-50% gradient of EtOAc in cyclohexane). Product-containing fractions were combined are combined and reduced under vacuum to afford methyl 2-chloro-3-(ethylsulfamoyl)-4-(trifluoromethoxy)benzoate (0.8 g, 2.4 mmol, 90%). 1H NMR (400 MHz, chloroform) δ ppm 1.10 - 1.21 (m, 3 H) 3.08 - 3.21 (m, 2 H) 3.93 - 4.01 (m, 3 H) 5.25 (t, 1 H) 7.38 - 7.46 (m, 1 H) 7.76 - 7.89 (m, 1 H).

### Step 5: Preparation of 2-chloro-3-(ethylsulfamoyl)-4-(trifluoromethoxy)benzoic acid

To a stirred solution of methyl 2-chloro-3-(ethylsulfamoyl)-4-(trifluoromethoxy)benzoate (0.8 g, 2 mmol) in tetrahydrofuran (10 mL) and water (2 mL) at room temperature was added lithium hydroxide monohydrate (0.3 g, 7 mmol). The mixture was stirred at room temperature overnight. The reaction was quenched by addition of 2 M aq. HCl (100 mL). The mixture was stirred at room temperature for a further 5 minutes. The mixture was then transferred to a separating funnel. EtOAc (100 mL) and brine (50 mL) were added and the phases were separated. The aqueous phase was extracted with EtOAc (100 mL). The combined organic phases were dried (MgSO₄), filtered and concentrated in vacuo to afford 2-chloro-3-(ethylsulfamoyl)-4-(trifluoromethoxy)benzoic acid as a beige solid (0.6 g, 1.7 mmol, 80%). 1H NMR (d4-methanol): 7.95 (d, 1H), 7.56 (m, 1H), 3.07 (m, 2H), 1.10 (m, 3H).

### Step 6: Preparation of 2-chloro-3-(ethylsulfamoyl)-N-(1-methyltetrazol-5-yl)-4-(trifluoromethoxy)benzamide

2-chloro-3-(ethylsulfamoyl)-4-(trifluoromethoxy)benzoic acid (600 mg, 1.7256 mmol), 1-methyltetrazol-5-amine (205 mg, 2.069 mmol) and 3-methylpyridine (8 mL) were stirred for 10 mins under a nitrogen atmosphere. Triethylamine (0.36 mL, 2.6 mmol) followed by 1-Methylimidazole (0.15 mL, 1.9 mmol) were added and the reaction mixture was stirred at RT for 30 min. The reaction mixture was then cooled to 0°C and thionyl chloride (0.25 mL, 3.4 mmol) was added, stirred at RT for 16 h. The reaction mixture was quenched with 2N HCl and stirred for 30 min. This is extracted with ethyl acetate x 2 and the combined organics are collected and reduced under vacuum. The crude material is purified by reverse phase chromatography (0-100% Acetonitrile in water with 0.1% formic acid). Product containing fractions were combined and concentrated by freeze-drying to afford 2-chloro-3-(ethylsulfamoyl)-N-(1-methyltetrazol-5-yl)-4-(trifluoromethoxy)benzamide as a white solid (0.23 g, 0.65 mmol, 38%). 1H NMR (400 MHz, d4-methanol) δ ppm 1.13 (t, 3 H) 3.10 (q, 2 H) 4.10 (s, 3 H) 7.61 - 7.69 (m, 1 H) 7.89 - 8.02 (m, 1 H).

**TABLE 1 - Examples of herbicidal compounds of the present invention.**

| **Compound Number** | **Structure** | **¹H NMR** |
|---|---|---|
| 1.001 | 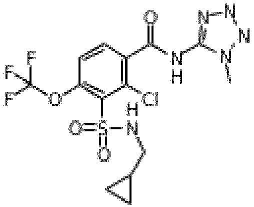 | (d4-methanol): 7.92 (d, 1H), 7.62 (d, 1H), 4.07 (s, 3H), 2.92 (d, 2H), 0.90-0.80 (m, 1H), 0.42-0.35 (m, 2H), 0.14-0.10 (m, 2H) |
| 1.002 | 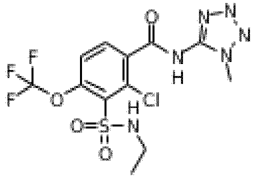 | (d4-methanol): 1.13 (t, 3 H) 3.10 (q, 2 H) 4.10 (s, 3 H) 7.61 - 7.69 (m, 1 H) 7.89 - 8.02 (m, 1 H) |
| 1.003 | 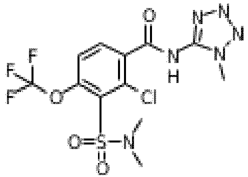 | (d4-methanol) 2.96 (s, 6 H) 4.10 (s, 3 H) 7.64 - 7.74 (m, 1 H) 7.92 - 8.03 (m, 1 H) |
| 1.004 | 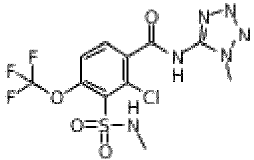 | (d4-methanol): 7.96 (d, 1H), 7.66 (d, 1H), 4.09 (s, 3H), 2.68 (s, 3H) |
| 1.005 | 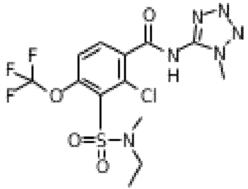 | |
| 1.006 | 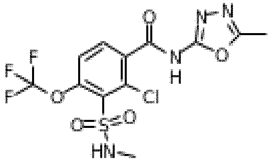 | |
| 1.007 | 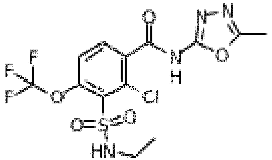 | |
| 1.008 | 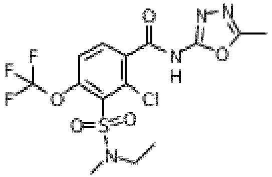 | |
| 1.009 | 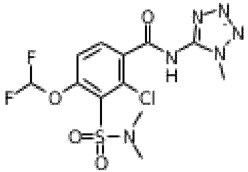 | (DMSO-d6): 11.91 (brs,1H), 8.02(d, 1H), 7.65-7.28(t, 1H), 7.53(d, 1H), 4.02(s, 3H), 2.89(s, 6H) |
| 1.010 | 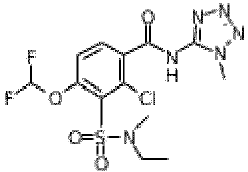 | (DMSO-d6): 11.88 (brs,1H), 7.99 (d, 1H), 7.63-7.27 (m,2H), 4.00 (s, 3H), 3.28 (q, 2H), 2.88 (s, 3H), 1.12 (t, 3H) |
| 1.011 | 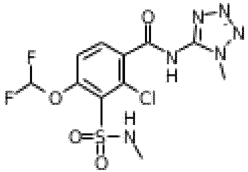 | |
| 1.012 | 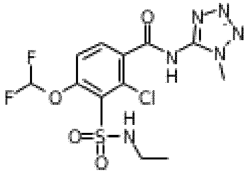 | (DMSO-d6): 11.91(brs,1H), 8.05(t,1H), 7.97(d, 1H), 7.55-7.14(m, 2H), 4.01 (s, 3H), 3.00 (q, 2H), 1.04 (t, 3H) |
| 1.013 | 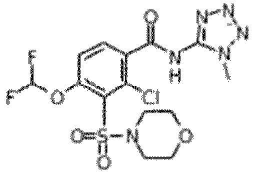 | (DMSO-d6): 11.92(brs,1H), 8.06 (d, 1H), 7.58-7.22(m,2H), 4.02(s,3H), 3.66(brs,4H), 3.26(brs,4H) |

### Biological Examples

Seeds of a variety of test species are sown in standard soil in pots (*Lolium perenne* (LOLPE), *Amaranthus retoflexus* (AMARE), *Abutilon theophrasti* (ABUTH), *Setaria faberi* (SETFA), *Echinochloa crus-galli* (ECHCG), *Zea mays* (ZEAMX)). After cultivation for one day under controlled conditions in a glasshouse (at 24/16°C, day/night; 14 hours light; 65 % humidity), the plants were sprayed with an aqueous spray solution derived from the formulation of the technical active ingredient in 0.6 ml acetone and 45 ml formulation solution containing 10.6% Emulsogen EL (Registry number 61791-12-6), 42.2% N-methyl pyrrolidone, 42.2% dipropylene glycol monomethyl ether (CAS RN 34590-94-8) and 0.2 % X-77 (CAS RN 11097-66-8).

The test plants were then grown in a glasshouse under controlled conditions in a glasshouse (at 24/16°C, day/night; 14 hours light; 65 % humidity) and watered twice daily. After 14 days, the test was evaluated (100 = total damage to plant; 0 = no damage to plant, NC = data not captured). The compounds were applied at 130g/ha unless otherwise stated.

**TABLE B1**

| Compound | POST Application | | | | | PRE Application | | | | |
|---|---|---|---|---|---|---|---|---|---|---|
| | AMARE | ABUTH | SETFA | ECHCG | ZEAMX | AMARE | ABUTH | SETFA | ECHCG | ZEAMX |
| 1.001 | 100 | 80 | 20 | 60 | 0 | 100 | 100 | 0 | 20 | 0 |
| 1.002 | 100 | 90 | 70 | 90 | 0 | 100 | 80 | 0 | 10 | 0 |
| 1.003 | 100 | 100 | 100 | 100 | 0 | 90 | 90 | 0 | 70 | 0 |
| 1.004 | 80 | 80 | 80 | 80 | 10 | 100 | 100 | 90 | 90 | 0 |
| 1.009 | 100 | NC | 90 | 90 | 10 | 100 | NC | 100 | 100 | 10 |
| 1.010 | 100 | NC | 90 | 100 | 10 | 100 | NC | 100 | 100 | 10 |

The experimental data shows that the compounds of the present invention provide significantly less damage to the maize (*ZEAMX*) plants whilst still providing good control of many weed species.

## Claims

1. A compound of Formula (I): 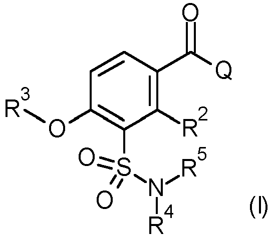
or an agronomically acceptable salt thereof,
wherein: -
Q is Q¹ or Q²; 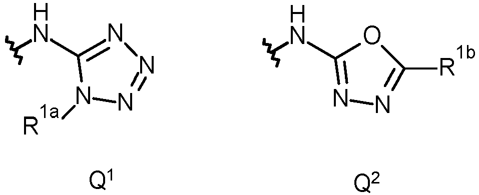
R^{1a} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R^{1b} is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is C₁-C₃haloalkyl;
R⁴ is selected from the group consisting of C₁-C₆alkyl-, C₁-C₆haloalkyl-, C₃-C₆cycloalkyl-, C₃-C₆cycloalkyl-C₁-C₃alkyl-, C₁-C₃alkoxy-C₁-C₃alkyl-, C₁-C₄haloalkoxy-C₁-C₄alkyl-, C₁-C₄alkoxy-C₁-C₄haloalkyl-, C₁-C₃alkyl-S(O)ₚC₁-C₃alkyl-, and cyano-C₁-C₆alkyl;
R⁵ is selected from the group consisting of hydrogen, C₁-C₆alkyl-, C₁-C₆haloalkyl and C₁-C₆cycloalkyl; or
R⁴ and R⁵ together with the nitrogen atom to which they are attached form an optionally substituted 5- or 6-membered saturated heterocycle; and
p is 0, 1 or 2.

2. A compound according to claim 1, wherein Q is Q¹.

3. A compound according to claim 1, wherein Q is Q².

4. A compound according to any one of the previous claims, wherein R^{1a} or R^{1b} are selected from the group consisting of methyl, ethyl and *n*-propyl.

5. A compound according to any one of the previous claims, wherein R² is selected from the group consisting of methyl, Cl, -CF₃ and -SO₂methyl.

6. A compound according to claim 5, wherein R² is Cl.

7. A compound according to any one of the previous claims, wherein R³ is -CF₃ or -CHF₂.

8. A compound according to any one of the previous claims, wherein R⁴ is selected from the group consisting of hydrogen, methyl, ethyl and cyclopropyl-CH₂-.

9. A compound according to any one of the previous clams, wherein R⁵ is hydrogen or methyl.

10. A herbicidal composition comprising a compound according to any one of the previous claims and an agriculturally acceptable formulation adjuvant.

11. A herbicidal composition according to claim 10, further comprising at least one additional pesticide.

12. A herbicidal composition according to claim 11, wherein the additional pesticide is a herbicide or herbicide safener.

13. A method of controlling weeds at a locus comprising application to the locus of a weed controlling amount of a composition according to any one of claims 10 to 12.

14. A compound of Formula (II) 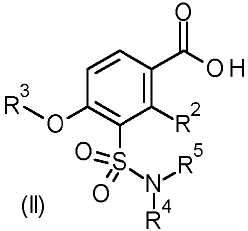 wherein R², R³, R⁴ and R⁵ are as defined in the compound of Formula (I) in any one of claims 1 to 10 above.

15. A compound of Formula (V) 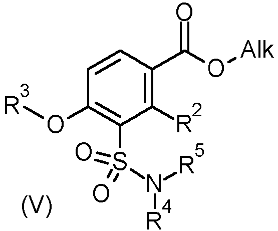 wherein "Alk" is C₁-C₆ alkyl and R², R³, R⁴ and R⁵ are as defined in the compound of Formula (I) in any one of claims 1 to 10 above.

16. Use of a compound of Formula (I) as defined in claim 1 as a herbicide.

## Patentansprüche

1. Verbindung der Formel (I): 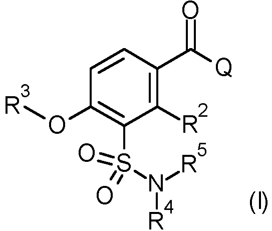
oder ein agronomisch unbedenkliches Salz davon, wobei: -
Q für Q¹ oder Q² steht; 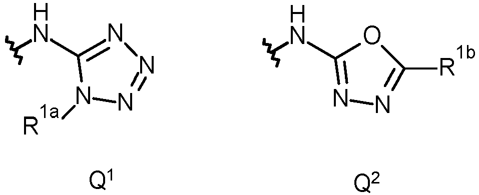
R^{1a} aus der Gruppe bestehend aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl- ausgewählt ist;
R^{1b} aus der Gruppe bestehend aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl- ausgewählt ist;
R² aus der Gruppe bestehend aus Halogen, C₁-C₆-Alkyl-, C₁-C₃-Alkoxy-, C₁-C₆-Halogenalkyl-, C₁-C₃-Halogenalkoxy- und -S(O)ₚ-C₁-C₆-Alkyl ausgewählt ist;
R³ für C₁-C₃-Halogenalkyl steht;
R⁴ aus der Gruppe bestehend aus C₁-C₆-Alkyl-, C₁-C₆-Halogenalkyl-, C₃-C₆-Cycloalkyl-, C₃-C₆-Cycloalkyl-C₁-C₃-alkyl-, C₁-C₃-Alkoxy-C₁-C₃-alkyl-, C₁-C₄-Halogenalkoxy-C₁-C₄-alkyl-, C₁-C₄-Alkoxy-C₁-C₄-halogenalkyl-, C₁-C₃-AlkylS(O)ₚC₁-C₃-alkyl- und Cyano-C₁-C₆-Alkyl ausgewählt ist;
R⁵ aus der Gruppe bestehend aus Wasserstoff, C₁-C₆-Alkyl-, C₁-C₆-Halogenalkyl und C₁-C₆-Cycloalkyl ausgewählt ist; oder
R⁴ und R⁵ zusammen mit dem Stickstoffatom, an das sie gebunden sind, einen 5- oder 6-gliedrigen gegebenenfalls gesättigten Heterocyclus bilden; und
p für 0, 1 oder 2 steht.

2. Verbindung nach Anspruch 1, wobei Q für Q¹ steht.

3. Verbindung nach Anspruch 1, wobei Q für Q² steht.

4. Verbindung nach einem der vorhergehenden Ansprüche, wobei R^{1a} oder R^{1b} aus der Gruppe bestehend aus Methyl, Ethyl und n-Propyl ausgewählt ist.

5. Verbindung nach einem der vorhergehenden Ansprüche, wobei R² aus der Gruppe bestehend aus Methyl, Cl, -CF₃ und -SO₂-Methyl ausgewählt ist.

6. Verbindung nach Anspruch 5, wobei R² für Cl steht.

7. Verbindung nach einem der vorhergehenden Ansprüche, wobei R³ für -CF₃ oder -CHF₂ steht.

8. Verbindung nach einem der vorhergehenden Ansprüche, wobei R⁴ aus der Gruppe bestehend aus Wasserstoff, Methyl, Ethyl und Cyclopropyl-CH₂- ausgewählt ist.

9. Verbindung nach einem der vorhergehenden Ansprüche, wobei R⁵ für Wasserstoff oder Methyl steht.

10. Herbizide Zusammensetzung, umfassend eine Verbindung nach einem der vorhergehenden Ansprüche und ein landwirtschaftlich unbedenkliches Formulierungshilfsmittel.

11. Herbizide Zusammensetzung nach Anspruch 10, ferner umfassend mindestens ein zusätzliches Pestizid.

12. Herbizide Zusammensetzung nach Anspruch 11, wobei es sich bei dem zusätzlichen Pestizid um ein Herbizid oder einen Herbizid-Safener handelt.

13. Verfahren zur Bekämpfung von Unkräutern an einem Standort, bei dem man eine unkrautbekämpfende Menge einer Zusammensetzung nach einem der Ansprüche 10 bis 12 auf den Standort ausbringt.

14. Verbindung der Formel (II) 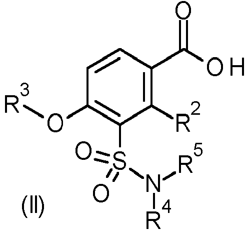 wobei R², R³, R⁴ und R⁵ wie in der Verbindung der Formel (I) in einem der Ansprüche 1 bis 10 oben definiert sind.

15. Verbindung der Formel (V) 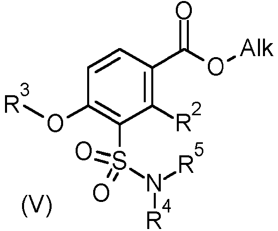 wobei "Alk" für C₁-C₆-Alkyl steht und R², R³, R⁴ und R⁵ wie in der Verbindung der Formel (I) in einem der Ansprüche 1 bis 10 oben definiert sind.

16. Verwendung einer Verbindung der Formel (I) gemäß Anspruch 1 als ein Herbizid.

## Revendications

1. Composé de formule (I) : 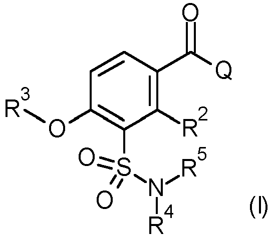
ou sel acceptable sur le plan agronomique correspondant,
Q étant Q¹ ou Q² ; 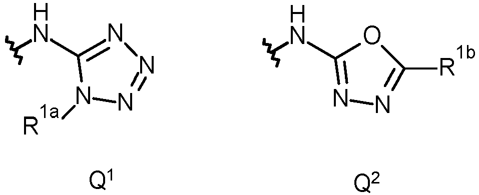
R^{1a} étant choisi dans le groupe constitué par C₁-C₄alkyle-, C₁-C₄halogénoalkyle-, C₁-C₄alcoxy-C₁-C₄alkyle- et C₁-C₄halogénoalcoxy-C₁-C₄alkyle- ;
R^{1b} étant choisi dans le groupe constitué par C₁-C₄alkyle-, C₁-C₄halogénoalkyle-, C₁-C₄alcoxy-C₁-C₄alkyle- et C₁-C₄halogénoalcoxy-C₁-C₄alkyle- ;
R² étant choisi dans le groupe constitué par halogène, C₁-C₆alkyle-, C₁-C₃alcoxy-, C₁-C₆ halogénoalkyle-, C₁-C₃halogénoalcoxy- et -S(O)ₚC₁-C₆alkyle ;
R³ étant C₁-C₃halogénoalkyle ;
R⁴ étant choisi dans le groupe constitué par C₁-C₆alkyle-, C₁-C₆halogénoalkyle-, C₃-C₆cycloalkyle-, C₃-C₆cycloalkyl-C₁-C₃alkyle-, C₁-C₃alcoxy-C₁-C₃alkyle-, C₁-C₄halogénoal-C₁-C₄alkyle-, C₁-C₄alcoxy-C₁-C₄halogénoalkyle-, C₁-C₃alkyl-S(O)ₚC₁-C₃alkyle-, et cyano-C₁-C₆alkyle ;
R⁵ étant choisi dans le groupe constitué par hydrogène, C₁-C₆alkyle-, C₁-C₆halogénoalkyle et C₁-C₆cycloalkyle ; ou R⁴ et R⁵ conjointement avec l'atome d'azote auquel ils sont fixés formant un hétérocycle saturé éventuellement substitué à 5 ou 6 chaînons ; et
p étant 0, 1 ou 2.

2. Composé selon la revendication 1, Q étant Q¹.

3. Composé selon la revendication 1, Q étant Q².

4. Composé selon l'une quelconque des revendications précédentes, R^{1a} ou R^{1b} étant choisi dans le groupe constitué par méthyle, éthyle et n-propyle.

5. Composé selon l'une quelconque des revendications précédentes, R² étant choisi dans le groupe constitué par méthyle, Cl, CF₃ et -SO₂méthyle.

6. Composé selon la revendication 5, R² étant Cl.

7. Composé selon l'une quelconque des revendications précédentes, R³ étant -CF₃ ou -CHF₂.

8. Composé selon l'une quelconque des revendications précédentes, R⁴ étant choisi dans le groupe constitué par hydrogène, méthyle, éthyle et cyclopropyl-CH₂-.

9. Composé selon l'une quelconque des revendications précédentes, R⁵ étant hydrogène ou méthyle.

10. Composition herbicide comprenant un composé selon l'une quelconque des revendications précédentes et un adjuvant de formulation acceptable sur le plan agricole.

11. Composition herbicide selon la revendication 10, comprenant en outre au moins un pesticide supplémentaire.

12. Composition herbicide selon la revendication 11, le pesticide supplémentaire étant un herbicide ou un phytoprotecteur herbicide.

13. Procédé de lutte contre des adventices au niveau d'un lieu comprenant une application sur le lieu d'une quantité de lutte contre des adventices d'une composition selon l'une quelconque des revendications 10 à 12.

14. Composé de formule (II) 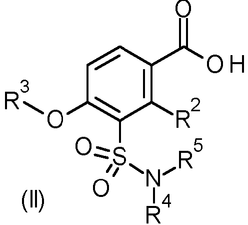 R², R³, R⁴ et R⁵ étant tels que définis dans le composé de formule (I) dans l'une quelconque des revendications 1 à 10 ci-dessus.

15. Composé de formule (V) 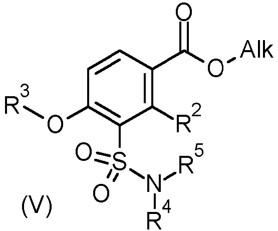 « Alk » étant C₁-C₆ alkyle et R², R³, R⁴ et R⁵ étant tels que définis dans le composé de formule (I) dans l'une quelconque des revendications 1 à 10 ci-dessus.

16. Utilisation d'un composé de formule (I) tel que défini dans la revendication 1 comme un herbicide.
